# EUROPEAN PATENT SPECIFICATION

(11) **EP 1 888 046 B1**
(45) Date of publication and mention of the grant of the patent: **17.02.2010**
(21) Application number: 06724260.2
(22) Date of filing: 12.04.2006
(51) Int. Cl.: A61K 9/51

(54) **PHARMACEUTICAL FORMULATIONS COMPRISING ACTIVE PHARMACEUTICAL PRINCIPLES ABSORBED ON TITANIUM DIOXIDE NANOPARTICLES**
AN TITANDIOXIDNANOPARTIKELN ABSORBIERTE PHARMAZEUTISCHE WIRKSTOFFE ENTHALTENDE PHARMAZEUTISCHE FORMULIERUNGEN
FORMULATIONS PHARMACEUTIQUES RENFERMANT DES PRINCIPES ACTIFS PHARMACEUTIQUES ABSORBÉS SUR DES NANOPARTICULES DE DIOXYDE DE TITANE

(30) Priority: 26.05.2005 US 684532 P
(43) Date of publication of application: 20.02.2008
(73) Proprietor: Flamma S.P.A., 24040 Chignolo D'Isola (BG) (IT)
(72) Inventor: MARRA, Alberto, I-83031 Ariano Irpino (IT); DONDONI, Alessandro, I-44100 Ferrara (IT); BIGNOZZI, Carlo, Alberto, I-44100 Ferrara (IT); CANEVOTTI, Renato, I-24049 Concorezzo (IT); NEGRISOLI, Gian Paolo, I-24129 Bergamo (IT); DISSETTE, Valeria, I-45011 Adria (IT)
(74) Representative: Minoja, Fabrizio
(86) International application number: PCT/EP2006/003348
(87) International publication number: WO 2006/125496

(56) References cited:
- WO-A1-03/032959
- DE-A1- 2 331 097
- FR-A1- 2 451 194
- US-A1- 2003 147 966
- US-A1- 2005 031 693

## Description

The present invention refers to pharmaceutical formulations comprising active pharmaceutical principles adsorbed on titanium dioxide nanoparticles.

The invention relates also to a process of adsorbing a drug on titanium dioxide nanoparticles through a direct interaction between the drug and titanium dioxide nanoparticles or via hydrophobic interactions assisted with fatty acids pre-adsorbed on the surface of titanium dioxide nanoparticles.

### Background of the invention

Nanoparticles have been studied extensively as particulate carriers in several pharmaceutical and medical fields (Sakuma S. et al., Adv Drug Del Rev 2001, 47: 21-37).

Titanium dioxide nanoparticles are known for instance from WO 01/42140, EP 1514845. In addition to the use as photocatalysts, carriers for catalysts and several other application in material science, titanium dioxide nanoparticles have been used as sunscreen formulations (US 2005249682) and for promoting plant growth (US 2005079977).

The use of titanium dioxide nanoparticles as carrier for drugs in pharmaceutical formulations has been reported in WO 03/032959, US 2003/147966 **and** FR 245194**.**

### Description of the invention

The invention relates to a process of adsorbing a drug on titanium dioxide nanoparticles through a direct interaction between the drug and the titanium dioxide nanoparticles or via a hydrophobic interaction with a fatty acid pre-adsorbed on the surface of titanium dioxide nanoparticles.

When adsorbed on titanium dioxide, the drug is no more, or only slightly, water soluble in the pH range 0-5 and can be desorbed from the titanium dioxide nanoparticles, becoming water soluble, in the pH range 6-14. This enables the preparation of new pharmaceutical forms which can be orally ingested allowing the drug release in the basic intestinal region with a minor release of the drug in the acidic gastrointestinal region, thus avoiding drug-induced gastro-esophageal injuries.

According to one embodiment of the invention, the drug is directly adsorbed on a titanium dioxide powder, where the dimension of the titanium dioxide nanoparticles have diameters in the 4-50 nm range (nm = nanometers).

According to an alternative embodiment of the invention, the drug is adsorbed on titanium dioxide nanoparticles precoated with fatty acids. In this case, the hydrophobic interaction between the long alkyl chains of the fatty acids, organized on the titanium dioxide nanoparticles, is weakened in the basic intestinal region.

Suitable fatty acids for coating the Titanium dioxide nanoparticles according to the invention include for instance C6-C30 linear or branched, optionally unsaturated, aliphatic or cycloaliphatic mono- or poly-carboxylic acids. Particularly preferred fatty acids are selected from Lauric, Mystic, Undecenoic, Pentadecanoic, Palmitic, Stearic, Arachidonic, Behenic and Lignoceric Acids.

The drug which may be advantageously used according to the invention is that of diphosphonic acids, including for instance alendronic, risedronic, pamidronic, chlodronic, neridronic, ibandronic, etidronic, mildronic, minodronic, zoledronic, cimadronic, tiludronic acids and salts thereof. This class of drugs, widely used in the treatment of osteoporosis and other bone diseases, is in fact know to have a poor gastric tolerability, as discussed for instance in WO 01/76577. This document discloses, as a possible solution to the above mentioned problem, the formulation of bisphosphonates with zwitterionic phospholipids.

The TiO₂ nanoparticles in the common form of Anatase, optionally containing different amounts of Rutile, are particularly preferred, being possible to produce powders with nanoparticle dimensions in the 4-50 nm range.

The adsorption of the drug or of the fatty acid which will then interact with a selected compound can be carried out in a polar or a non polar solvent both protic or aprotic, independently from the protonation degree of the carboxylic, phosphonic or boronic functions. It is preferred that the drug contains such functional groups in their fully protonated form.

Suitable solvents include for instance alcohols, acetone, acetonitrile, water or mixtures thereof.

The adsorption of a water-soluble drug on the nanoparticles is carried out by mixing an aqueous solution of the drug with the nanoparticles and stirring the obtained suspension for a period of time from 6 to 72 hours, at temperatures ranging from 20 to 40°C.

The pre-coating of titanium dioxide nanoparticles with fatty acids is carried out by mixing a solution of a fatty acid in a solvent, for instance methanol or ethanol, with the nanoparticles and stirring the obtained suspension for a period of time from 6 to 72 hours, at temperatures ranging from 20 to 40°C.

From about 20 mg to about 100 mg of fatty acids may be accordingly adsorbed on 1.00 g of titanium dioxide nanoparticles. Desorption experiments at different pH showed no appreciable release of fatty acids from the nanocrystalline substrate. This fact allows to rule out that the adsorbed fatty acids may trap on the surface of TiO₂ the drug with its subsequent release after fatty acid desorption.

The adsorption of a water-insoluble or poorly water soluble drug on the nanoparticles is carried out by mixing a solution of the drug in an organic solvent with the nanoparticles pre-coated with fatty acids and stirring the obtained suspension for a period of time from 6 to 72 hours, at temperatures ranging from 20 to 40°C.

The weight ratio of titanium dioxide nanoparticles to a selected drug is not critical and may range within wide limits. Anyhow, it will generally be from 10 to 100 parts by weight of titanium dioxide nanoparticles per part of drug.

The titanium dioxide nanoparticles loaded with a selected drug may be formulated into suitable oral administration forms, optionally in admixture with usual excipients. Examples of suitable formulations include tablets, soft or hard gelatine capsules, granules, powders, pills.

The amount of the titanium dioxide particles will depend of course on the kind of adsorbed drug and will be easily determined by the skilled person according to the guidance reported in the following examples and on the basis of simple routine experiments.

The adsorbed drug is delivered to the intestinal region without appreciable release in the gastroesophageal region.

The invention is illustrated in more detail by the following examples.

### EXAMPLE 1 - Sodium Risedronate

### Adsorption on TiO₂

The electronic absorption spectra of both protonated and deprotonated Sodium Risedronate are characterized by an intense band in the UV region that can be confidently assigned to π-π* transitions localized on the pyridine ring.

To a stirred solution of Sodium Risedronate (625 mg) in deionized water (100 ml) increasing amounts of nanocrystalline TiO₂ were added (ca. 20 nm nanoparticle size). The absorption spectra show that the amount of drug which can be adsorbed on the surface of the nanocrystalline substrate reaches the value of 31 %. This limit probably corresponds to a total surface coverage of the nanoparticles.

190 mg of Sodium Risedronate were adsorbed at 25°C on 3.00 g of TiO₂ nanoparticles having an average diameter of 20-25 nm.

### Desorption from TiO₂

Desorption of Sodium Risedronate from TiO₂ at 25°C was studied in aqueous solution at different pH values. The absorption spectra of the solutions at different pH obtained after a 2 h stirring show that the amount of Risedronate released to the solution increases with pH and that the amount of Risedronate released after 30 min at pH = 8 is comparable to that released after 2 hours, i.e. 35% of the initial concentration on TiO₂.

### Desorption of Sodium Risedronate from TiO₂ at pH 9, 36°C

The desorption as function of time of Sodium Risedronate from TiO₂ was studied in aqueous solution at pH 9 and at 36°C.

### Samples preparation

To an aqueous solution containing 625 mg of Sodium Risedronate in 100 ml of deionized water 3.00 g of TiO₂ was added. The suspension was stirred for 72 h at room temperature and then centrifuged at 4000 rpm for 10 min. The solid was suspended twice in a pH I HCI solution, stirred for 20 min and centrifuged. The solid was finally dried at 40°C under vacuum.

The spectrophotometric analysis showed that 190 mg of Sodium Risedronate were adsorbed by 3.00 g of TiO₂.

Nine separate 30 mg portions of TiO₂ adsorbed with Sodium Risedronate were suspended in 10 ml of pH 9 NaOH solutions and left stirring for different times in the range 1-120 min. During drug desorption the pH of the suspension was kept at the original value by small addition of NaOH solution. In all cases the concentration of desorbed drug was corrected for volume changes.

The results obtained indicate that, after 1 minute, 25% of the adsorbed Sodium Risedronate was immediately desorbed. The amount of drug released increased, almost linearly, up to a value of 36% in the next 60 min, followed by a constant release of 36% at longer times.

Based on desorption and kinetic studies it is possible to conclude that 250 mg of TiO₂ carrying 16 mg of Sodium Risedronate should allow to release in the intestine 6-9 mg of the drug with negligible release in the gastroesophageal region (the daily dose is 5-35 mg; the oral bioavailability is 0.63%).

### Example 2-Sodium Alendronate

A NMR-based method was developed since alendronate lacks the chromophoric group of risedronate. The NMR experiments were carried out in H₂O instead of D₂O (or other deuterated solvents) simply using the aqueous solutions obtained after removal of TiO₂ by centrifugation without concentration or further treatment. Only a small amount of C₆D₆ in a sealed capillary tube (reusable) inserted into the NMR tube was required to achieve the field lock. In the broad-band proton decoupled ³¹P-NMR spectrum of sodium alendronate in H₂O at 25°C the signal corresponding to the two phosphonate groups appeared as a singlet at 19 ppm. To determine the concentration of sodium alendronate in aqueous solutions, a known amount of an internal standard was added before each NMR measurements, namely 1,3,5-triaza-7-phospha-adamantane, a water-soluble phosphine. Due to the different oxidation state of the phosphorous atoms in these two classes of compounds (R₃P *vs*. R-P(O)(OH)₂), significant differences in the relaxation times and therefore in the corresponding signal integrals were found. However, upon careful adjustment of the NMR acquisition parameters, a very good agreement between the signal integrals and the actual molar ratio of known mixtures of sodium alendronate (two P atoms per molecule) and 1,3,5-triaza-7-phospha-adamantane (one P atom per molecule) in water was observed.

### Adsorption on TiO₂

TiO₂ (25 nm, 1.00 g) was added to a solution of Sodium Alendronate trihydrated (207 mg, 0.64 mmol) in H₂O (50 ml) and the resulting suspension (pH 4.5) was vigorously stirred in the dark at 25°C for 48 h, then centrifuged (4,000 rpm, 5 min). The cloudy supernatant was recovered, treated with solid NaCI (ca. 3 g), kept at room temperature for 2 h, and centrifuged (4,000 rpm, 5 min). To the resulting solution was added 1,3,5-triaza-7-phospha-é adamantane and the concentration of residual sodium alendronate was estimated by ³¹P-NMR as described above.

The analysis showed that 71 mg (34% of the initial amount) of alendronate was adsorbed. We found that after 24 h stirring (instead of 48 h) at 25°C, 66 mg (32% of the initial amount) of alendronate was adsorbed. Upon repeated experiments (48 h, 25°C) adsorption range was 26-34%.

### Desorption from TiO₂

Sodium Alendronate trihydrated was adsorbed on TiO₂ as described above. After centrifugation, the recovered solid was dried at 40°C/0.1 mar for 6 h. A suspension of this solid in 50 ml of aqueous HCI (pH 1.0) or NaOH (pH 9.0) was vigorously stirred at the stated temperature in a nitrogen atmosphere. The pH of the basic solution was constantly monitored and maintained at the initial level by addition of 0.05 M NaOH.

Desorption at pH 1.0, 2 h stirring at 25°C: sodium alendronate was not detected by NMR analysis.
Desorption at pH 9.0, 30 min stirring at 25°C: 8%
Desorption at pH 9.0, 2 h stirring at 25°C: 12%
Desorption at pH 9.0, 6 stirring at 37°C: 22%

Upon repeated experiments (pH 9.0, 6 h, 37°C) a 16-22% desorption range was found.

250 mg of TiO₂ carrying 16 mg of Alendronate should allow the release to the intestine of 4 mg of the drug.

## Claims

1. Oral pharmaceutical formulations comprising as the active ingredient an alendronic, risedronic, pamidronic, chlodronic, neridronic, ibandronic, etidronic, mildronic, minodronic, zoledronic, cimadronic, tiludronic acids and salts thereof adsorbed on titanium dioxide nanoparticles.

2. Formulations according to **claim 1** wherein the TriO₂ nanoparticles consist of Anatase, optionally containing different amounts of Rutile, having nanoparticle dimensions from 4 to 50 nm.

3. Formulations according to **claims 1 or 2** in form of tablets, capsules, granules, powders, pills.

4. Titanium dioxide nanoparticles as carriers for selective pH dependent delivery in the gastro-intestinal tract of **bisphosphonates drugs of claim 1.**

5. A process for adsorbing a **bisphosphonate drug of claim 1** on titanium dioxide nanoparticles which comprises the mixing of a solution of the drug in water or organic solvent with the nanoparticles, either as such or pre-coated with fatty acids, and stirring the obtained suspension for a period of time from 6 to 72 hours, at temperatures ranging from 20 to 40°C.

## Patentansprüche

1. Orale pharmazeutische Formulierungen, die als Wirkstoff eine Alendron-, Risedron-, Pamidron-, Chlodron-, Neridron-, Ibandron-, Etidron-, Mildron-, Minodron-, Zoledron-, Cimadron-, Tiludronsäure und Salze davon umfassen, die auf Titandioxid-Nanopartikeln adsorbiert sind.

2. Formullerungen nach Anspruch 1, wobei die TiO₂-Nanopartikel aus Anatas bestehen, optional verschiedene Mengen an Rutil enthalten, und Nanopartikelgrößen von 4 bis 50 nm aufweisen,

3. Formulierungen nach den Ansprüchen 1 oder 2, in der Form von Tabletten, Kapseln, Granulat, Pulvern, Pillen.

4. Titandioxid-Nanopartikel als Träger für die selektive pH-abhängige Abgabe von Bisphosphcanat-Arzneien nach Anspruch 1 im Magen-Darm-Trakt.

5. Ein Verfahren zur Adsorption einer Bisphosphonat-Arznei nach Anspruch 1 auf Titandioxid-Nanopartikeln, das das Mischen einer Lösung der Arznei in Wasser oder einem organischen Lösungsmittel mit den Nanopartikeln, entweder als solche oder mit Fettsäuren vorbeschichtet, und das Rühren der erhaltenen Suspension für eine Zeitdauer von 6 bis 72 Stunden bei Temperaturen im Bereich von 20 bis 40°C umfasst.

## Revendications

1. Formulations pharmaceutiques orales comprenant en tant qu'agent actif un parmi les acides alendronique, risédronique, pamidronique, clodronique, néridronique, ibandronique, étidronique, mildronique, minodronique, zolédronique, cimadronique, tiludronique et leurs sels adsorbé sur des nanoparticules de dioxyde de titane.

2. Formulations selon la revendication 1 dans lesquelles les nanoparticules de TiO₂ sont constituées d'anatase, contenant éventuellement différentes quantités de rutile, ayant des dimensions de nanoparticules de 4 à 50 nm.

3. Formulations selon la revendication 1 ou 2 sous forme de comprimés, capsules, granules, poudres, pilules.

4. Nanoparticules de dioxyde de titane en tant que supports pour la délivrance pH-dépendante sélective dans le tractus gastro-intestinal des médicaments diphosphonates selon la revendication 1.

5. Procédé pour adsorber un médicament diphosphonate selon la revendication 1 sur des nanoparticules de dioxyde de titane qui comprend le mélange d'une solution du médicament dans l'eau ou un solvant organique avec les nanoparticules, soit telles quelles soit pré-enrobées avec des acides gras, et l'agitation de la suspension obtenue pendant une durée de 6 à 72 heures, à des températures allant de 20 à 40°C.
